# EUROPEAN PATENT APPLICATION

(11) **EP 2 108 305 A1**
(43) Date of publication of application: **14.10.2009**
(21) Application number: 08703785.9
(22) Date of filing: 24.01.2008
(51) Int. Cl.: A61B 1/04, H04N 9/04, H04N 9/09

(54) **HEAD SEPARATED CAMERA AND CAMERA HEAD**

(30) Priority: 29.01.2007 JP 2007017935
(71) Applicant: Panasonic Corporation, Kadoma-shi Osaka 571-8501 (JP)
(72) Inventor: KOBAYASHI, Katsuhiro, Shiromi 2-chome, Chuo-ku, Osaka-shi, Osaka 540-6207 (JP)
(74) Representative: Kügele, Bernhard
(86) International application number: PCT/JP2008/050957
(87) International publication number: WO 2008/093589

(57) **Abstract**

A camera head (2) of a head separated camera (1) includes an adjustment value storage unit (9) that stores black balance adjustment values OBR, OBG and OBB for three image pickup devices (5) and a black balance adjusting circuit (8) that adjusts the black balance based on the black balance adjustment values OBR, OBG and OBB so as to make the black levels of the video signals approximately equal to each other. The video signals adjusted in black balance by the black balance adjusting circuit (8) are transmitted from the camera head (2) to a camera control unit (3). Thus, there is provided a head separated camera that does not have to readjust the black balance even if the combination of the camera head, a camera cable and the camera control unit is changed.

## Description

### Technical Field

The present invention relates to a head separated camera. In particular, it relates to a camera head capable of adjusting the black balance.

### Background Art

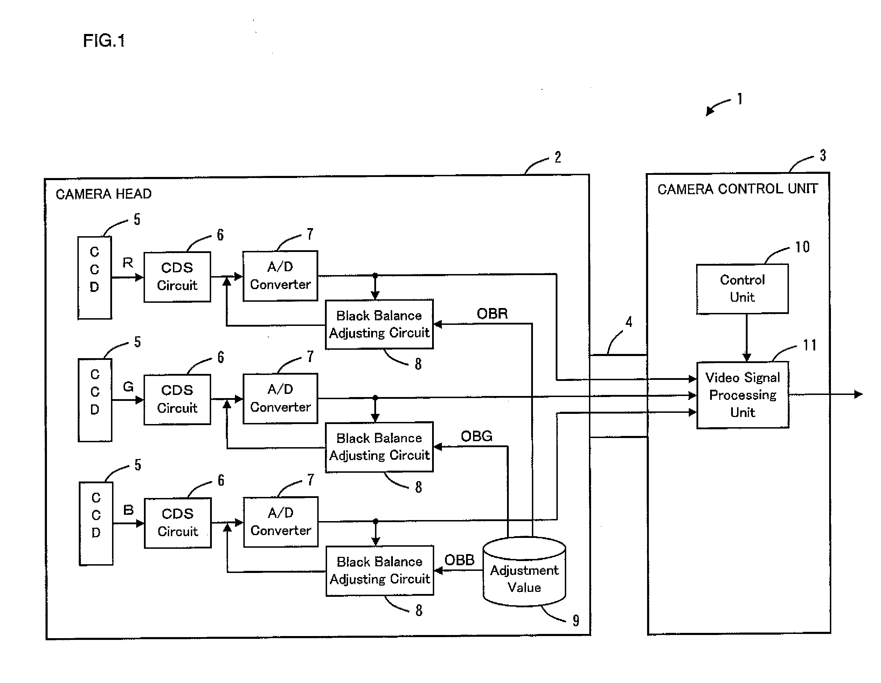

A known conventional head separated camera has a camera head incorporating an image pickup device, such as a CCD, removably attached to a camera control unit for controlling the operation of the camera head by a camera cable. The head separated camera is used as an endoscope for medical or industrial use, for example. For example, Japanese Patent Laid-Open No. 11-197103 (p. 2 - 3 and Figure 1) discloses the head separated camera of this type.

When the conventional head separated camera performs black balance adjustment, an analog video signal produced by the CCD with the camera head shielded from light is transmitted as a black level signal from the camera head to the camera control unit through the camera cable. Alternatively, an analog video signal produced by an optical black part (OB part) of the CCD is transmitted as a black level signal from the camera head to the camera control unit through the camera cable. Then, the black balance adjusting circuit in the camera control unit adjusts the black balance based on the black level signal.

However, there are various types of camera heads available for the conventional head separated camera, and the characteristics of the CCD, the CDS circuit or the like vary with the camera head. In addition, there are a wide variety of camera cables of different lengths, and the characteristics, such as the attenuation factor, vary with the camera cable. In addition, there are various types of camera control units, and the characteristics of the processor or the like vary with the camera control unit. Therefore, for the conventional head separated camera, the black balance adjusting circuit in the camera control unit has to readjust the black balance each time the combination of the camera head, the camera cable and the camera control unit is changed.

### Disclosure of the Invention

The present invention is devised in view of such circumstances. An object of the present invention is to provide a head separated camera that does not require black balance readjustment when the combination of the camera head, the camera cable and the camera control unit is changed.

An aspect of the present invention is a head separated camera. The head separated camera is a head separated camera in which a camera head is removably attached to a camera control unit that controls the camera head, the camera head comprises a plurality of image pickup devices, an adjustment value storage unit that stores a black balance adjustment value set to be specific to the camera head as a black balance adjustment value for each of the plurality of image pickup devices, and a black balance adjusting unit that adjusts the black balance of video signals produced by the plurality of image pickup devices based on the black balance adjustment values so as to make the black levels of the video signals approximately equal to each other, and the video signals adjusted in black balance by the black balance adjusting unit are transmitted from the camera head to the camera control unit.

Another aspect of the present invention is a camera head for a head separated camera. The camera head for a head separated camera comprises a plurality of image pickup devices, an adjustment value storage unit that stores a black balance adjustment value set to be specific to the camera head as a black balance adjustment value for each of the plurality of image pickup devices, and a black balance adjusting unit that adjusts the black balance of video signals produced by the plurality of image pickup devices based on the black balance adjustment values so as to make the black levels of the video signals approximately equal to each other.

As described later, the present invention has other aspects. Therefore, the disclosure of the present invention herein is not intended to limit the scope of the present invention described and claimed herein but to provide some of the aspects of the present invention.

### Brief Description of the Drawings

Figure 1 is a block diagram showing a head separated camera according to an embodiment of the present invention;
Figure 2 is a perspective view of the head separated camera according to the embodiment;
Figure 3 is a perspective view of a head separated camera with another camera head;
Figure 4 is a block diagram showing a head separated camera according to another embodiment; and
Figure 5 is a block diagram showing a head separated camera according to another embodiment.

### Description of Symbols

- 1: head separated camera
- 2: camera head
- 3: camera control unit
- 4: camera cable
- 5: image pickup device
- 7: A/D converter
- 8: black balance adjusting circuit
- 9: adjustment value storage unit

### Best Mode for Carrying Out the Invention

In the following, the present invention will be described in detail. However, the following detailed description and the accompanying drawings are not intended to limit the scope of the present invention. The scope of the present invention is defined only by the accompanying claims.

A head separated camera according to the present invention is a head separated camera in which a camera head is removably attached to a camera control unit that controls the camera head, the camera head comprises a plurality of image pickup devices, an adjustment value storage unit that stores a black balance adjustment value set to be specific to the camera head as a black balance adjustment value for each of the plurality of image pickup devices, and a black balance adjusting unit that adjusts the black balance of video signals produced by the plurality of image pickup devices based on the black balance adjustment values so as to make the black levels of the video signals approximately equal to each other, and the video signals adjusted in black balance by the black balance adjusting unit are transmitted from the camera head to the camera control unit.

According to this configuration, the adjustment value storage unit in the camera head stores the black balance adjustment value for each of the plurality of image pickup devices. The black balance adjustment values are set to be specific to the camera head. The black balance adjusting unit in the camera head uses the black balance adjustment values to adjust the black balance of the video signals from the plurality of image pickup devices. In this case, the black balance is adjusted so as to make the black levels of the video signals approximately equal to each other. As described above, the camera head has the black balance adjustment function, and the video signals adjusted in black balance are transmitted from the camera head to the camera control unit. Therefore, even if the camera head is replaced with a different type, the camera control unit does not have to readjust the black balance.

Furthermore, for the head separated camera according to the present invention, the black balance adjustment values may include a reference adjustment value for black balance derived from the video signal from a reference image pickup device, which is one of the plurality of image pickup devices, and a relative correction parameter for correcting the gap in black balance between the reference image pickup device and each of the plurality of image pickup devices, and the black balance adjusting unit may adjust the black balance of the video signals based on the reference adjustment value and the relative correction parameters so as to make the black levels of the video signals approximately equal to each other.

According to this configuration, the adjustment value storage unit in the camera head stores the reference adjustment value for black balance and the relative correction parameters. The black balance adjusting unit in the camera head uses the reference adjustment value and the relative correction parameters to adjust the black balance of the video signals from the plurality of image pickup devices. For example, in the case where the camera head has three image pickup devices for the R signal, the G signal and the B signal, the reference adjustment value for the image pickup device for the G signal (reference image pickup device) and the relative correction parameter for the image pickup device for the R signal are used to correct the gap in black balance between the image pickup device for the R signal and the image pickup device for the G signal. And the reference adjustment value for the image pickup device for the G signal and the relative correction parameter for the image pickup device for the B signal are used to correct the gap in black balance between the image pickup device for the B signal and the image pickup device for the G signal. In this way, the black balance can be adjusted so as to make the black levels of the R, G and B video signals approximately equal to each other.

For the head separated camera according to the present invention, the camera head may further comprise an analog-to-digital converting unit that converts the video signals from the analog form to the digital form, and the video signals may be digitally transmitted from the camera head to the camera control unit.

According to this configuration, the analog-to-digital converting unit in the camera head converts the analog video signal into the digital video signal. Then, the resulting digital video signal is digitally transmitted from the camera head to the camera control unit. Thus the video signals can be more easily transmitted from the camera head to the camera control unit.

A camera head according to the present invention is a camera head for a head separated camera, comprising a plurality of image pickup devices, an adjustment value storage unit that stores a black balance adjustment value set to be specific to the camera head as a black balance adjustment value for each of the plurality of image pickup devices, and a black balance adjusting unit that adjusts the black balance of video signals produced by the plurality of image pickup devices based on the black balance adjustment values so as to make the black levels of the video signals approximately equal to each other.

According to this configuration, as described above, the camera head has the black balance adjustment function, and the video signals adjusted in black balance are transmitted from the camera head to the camera control unit. Therefore, the black balance does not have to be readjusted in the camera control unit when the combination of the camera head, the camera cable and the camera control unit is changed.

According to the present invention, since the camera head has the black balance adjustment value storage unit and the black balance adjusting unit and therefore has the black balance adjustment function, the black balance does not have to be readjusted when the combination of the camera head, the camera cable and the camera control unit is changed.

In the following, a head separated camera according to an embodiment of the present invention will be described with reference to the drawings. With regard to this embodiment, a head separated camera used as an endoscope for medical or industrial use will be described as an example.

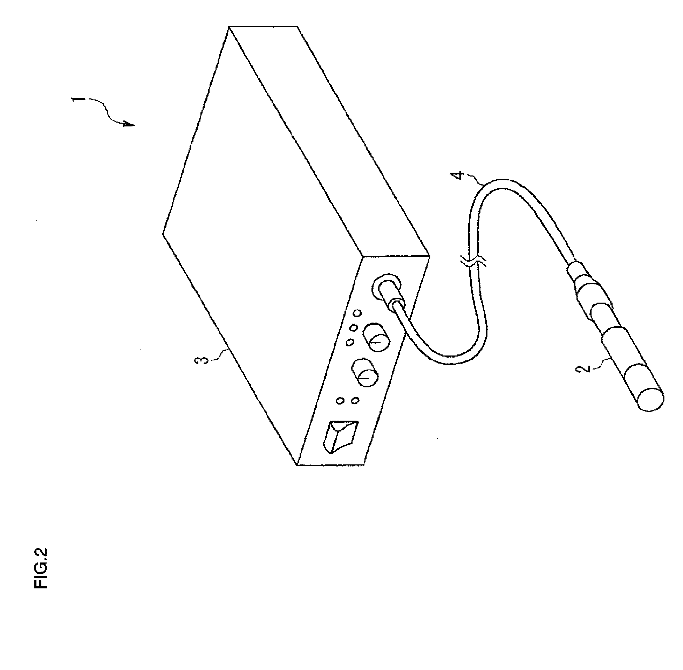

The head separated camera according to this embodiment will be described with reference to Figures 1 to 3. Figure 1 is a block diagram showing a configuration of the head separated camera. Figure 2 is a perspective view of the head separated camera.

As shown in Figures 1 and 2, a head separated camera 1 has a camera head 2 and a camera control unit 3 that controls the camera head 2. The camera head 2 is connected to the camera control unit 3 by a camera cable 4, and the camera cable 4 is removably plugged into the camera control unit 3. That is, the camera head 2 is removably attached to the camera control unit 3. In other words, the camera head 2 is replaceable.

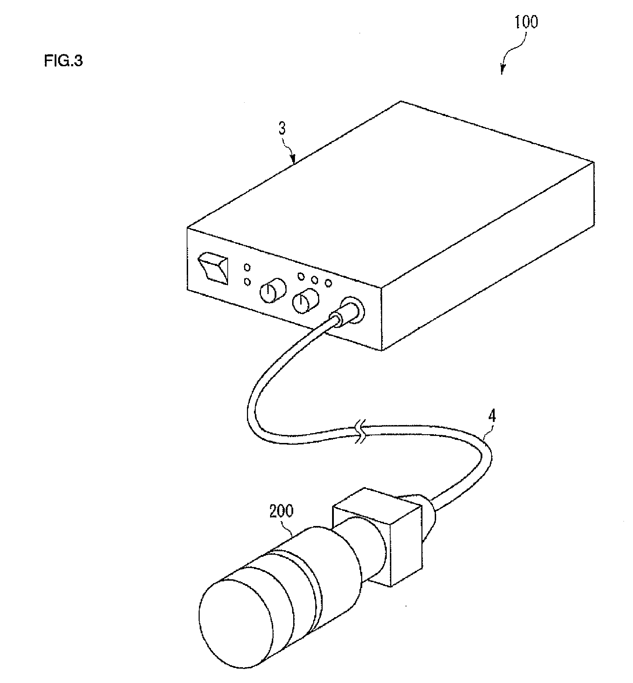

Figure 3 is a perspective view of another head separated camera with another camera head attached thereto. Figure 3 shows a head separated camera 100 having a large camera head 200 instead of the small camera head 2 shown in Figure 2. Various kinds of camera heads can be used depending on the application or required performance, for example. As shown in Figure 3, depending on the application of the head separated camera 100, for example, an appropriate type of camera head is used. Although not shown, the camera cable 4 can be of various lengths. And depending on the application of the head separated camera, a camera cable having an appropriate length is used.

In the following, an arrangement of the camera head 2 and the camera control unit 3 will be described. First, components of the camera head 2 will be described with reference to Figure 1.

As shown in Figure 1, the camera head 2 has three image pickup devices 5. The image pickup devices 5 are solid-state image pickup devices, such as CCDs, and are provided for the R signal, the G signal and the B signal, respectively. That is, one of the three image pickup devices 5 is provided for the R signal, another is provided for the G signal, and the remaining one is provided for the B signal, and thus, the head separated camera 1 can be regarded as the so called 3-CCD camera.

The camera head 2 has CDS circuits 6 for performing correlated double sampling disposed subsequent to the image pickup devices 5. In addition, A/D converters 7 for performing analog-to-digital conversion are disposed subsequent to the CDS circuits 6. The A/D converter 7 herein corresponds to an analog-to-digital converting unit according to the present invention.

The camera head 2 has black balance adjusting circuits 8 disposed subsequent to the A/D converters 7, and the black balance adjusting circuit 8 is constituted by an optical black lamp circuit, for example. Using black balance adjustment values described later, the black balance adjusting circuits 8 adjust the black level of each of the video signals (R signal, G signal and B signal) produced by the three image pickup devices 5 so as to make the black levels of the video signals approximately equal to each other. The black balance adjusting circuit 8 herein corresponds to a black balance adjusting unit according to the present invention.

The camera head 2 further has an adjustment value storage unit 9, which is constituted by a memory, such as an EEPROM. The adjustment value storage unit 9 stores black balance adjustment values OBR, OBG and OBB for the three image pickup devices 5. The adjustment value storage unit 9 herein corresponds to an adjustment value storage unit according to the present invention.

The black balance adjustment values OBR, OBG and OBB vary with the camera head 2 or, in other words, are specific to each individual camera head 2. The black balance adjustment values OBR, OBG and OBB are previously measured when the camera head 2 is shipped from the factory, for example, and stored in the adjustment value storage unit 9 in the camera head 2. In other words, the black balance adjustment values OBR, OBG and OBB are set for each individual camera head 2.

According to this embodiment, the adjustment value storage unit 9 stores the black balance adjustment value OBG received from the image pickup device 5 for the G signal, a relative correction parameter KR that corrects the gap in black balance between the image pickup device 5 for the R signal and the image pickup device for the G signal, and a relative correction parameter KB that corrects the gap in black balance between the image pickup device 5 for the B signal and the image pickup device for the G signal. The image pickup device 5 for the G signal herein corresponds to a reference image pickup device according to the present invention, and the black balance adjustment value OBG for the image pickup device 5 herein corresponds to a reference adjustment value according to the present invention.

In this case, the reference adjustment value OBG, the relative correction parameters KR and KB, and the black balance adjustment values of the image pickup devices 5 relate to each other according to the following formulas.
OBR = OBG × KR
OBG = OBG
OBB = OBG × KB

When the gap in black balance among the three image pickup devices 5 (difference in black balance characteristics among the CCDs) falls within an allowable range, the adjustment value storage unit 9 in the camera head 2 may be omitted, and a prescribed value may be set in the black balance adjusting circuits 8.

Next, components of the camera control unit 3 will be described with reference to Figure 1.

As shown in Figure 1, the camera control unit 3 has a control unit 10 constituted by a microcomputer or the like and a video signal processing unit 11 constituted by a digital signal processor (DSP) or the like. The video signal processing unit 11 performs various kinds of video signal processings on the video signal transmitted from the camera head 2.

The camera cable 4 digitally transmits the video signal from the camera head 2 to the camera control unit 3. According to this embodiment, the video signal is digitally transmitted from the camera head 2 to the camera control unit 3 by low voltage differential signaling (LVDS) or the like. In this case, the video signal is multiplexed with a control signal for a switch on the camera head 2 or memory data, for example. Furthermore, the camera cable 4 also bidirectionally transmits a clock signal or a shutter control signal from the control unit 10 of the camera control unit 3, for example. Furthermore, the camera cable 4 includes a buffer circuit that prevents attenuation of the amplitude of the digital signal.

Next, a black balance adjustment operation by the head separated camera 1 configured as described above will be described.

### (Setting of black balance adjustment value)

For the head separated camera 1 according to this embodiment of the present invention, the black balance adjustment values OBR, OBG and OBB are previously set when the camera 1 is shipped from the factory, for example. For example, shooting is performed with the camera head 2 shielded from light, and the three image pickup devices 5 output their respective video signals (black level signals).

The video signals (black level signals) output from the image pickup devices 5 are input to the respective CDS circuits 6, which remove noise from the video signals by correlated double sampling. Then, the video signals are output from the CDS circuits 6 and input to the A/D converters 7, which perform analog-to-digital conversion on the video signals.

The video signals having been subject to analog-to-digital conversion are input to the black balance adjusting circuits 8, which perform black balance adjustment. At this time, the black balance adjustment values OBR, OBG and OBB are measured and stored in the adjustment value storage unit 9. In this way, the black balance adjustment values OBR, OBG and OBB are set for each individual camera head 2.

### (Black balance adjustment in video shooting)

When the head separated camera 1 according to this embodiment is used for video shooting, the three image pickup devices 5 output their respective video signals (R signal, G signal and B signal).

The video signals (R signal, G signal and B signal) output from the image pickup devices 5 are input to the respective CDS circuits 6, which remove noise from the video signals by correlated double sampling. Then, the video signals are output from the CDS circuits 6 and input to the A/D converters 7, which perform analog-to-digital conversion on the video signals.

The video signals having been subject to analog-to-digital conversion are input to the black balance adjusting circuits 8, which perform black balance adjustment. Then, using the previously set black balance adjustment values OBR, OBG and OBB, the black balance is adjusted so as to make the black levels of the video signals approximately equal to each other.

The video signals adjusted in black balance are digitally transmitted from the camera head 2 to the camera control unit 3 through the camera cable 4. Then, the video signal processing unit 11 in the camera control unit 3 performs various kinds of video signal processings, and the resulting signals are output as output video signals.

For the head separated camera 1 according to this embodiment of the present invention, the camera head 2 has the black balance adjustment value storage unit 9 and the black balance adjusting circuit 8. Thus, the camera head 2 has the black balance adjustment function, and therefore, the black balance does not have to be readjusted when the combination of the camera head 2, the camera cable 4 and the camera control unit 3 is changed.

More specifically, according to this embodiment, the adjustment value storage unit 9 in the camera head 2 stores the black balance adjustment value for each of the three image pickup devices 5. The black balance adjustment values are set for each individual camera head 2. The black balance adjusting circuit 8 in the camera head 2 uses the black balance adjustment values to adjust the black balance of the video signals from the three image pickup devices 5. In this case, the black balance is adjusted so as to make the black levels of the video signals from the three image pickup devices 5 approximately equal to each other. In this way, the camera head 2 has the black balance adjustment function, and the video signals adjusted in black balance are transmitted from the camera head 2 to the camera control unit 3. That is, analog fluctuations can be compensated for within the camera head 2. Therefore, the black balance does not have to be readjusted when the combination of the camera head 2, the camera cable 4 and the camera control unit 3 is changed.

Furthermore, according to this embodiment, the adjustment value storage unit 9 in the camera head 2 stores the reference adjustment value for black balance and the relative correction parameters. The black balance adjusting circuit 8 in the camera head 2 uses the reference adjustment value and the relative correction parameters to adjust the black balance of the video signals from the three image pickup devices 5. For example, in the case where the camera head 2 has three image pickup devices 5 for the R signal, the G signal and the B signal, the reference adjustment value for the image pickup device 5 for the G signal (reference image pickup device) and the relative correction parameter for the image pickup device 5 for the R signal are used to correct the gap in black balance between the image pickup device 5 for the R signal and the image pickup device 5 for the G signal. And the reference adjustment value for the image pickup device 5 for the G signal and the relative correction parameter for the image pickup device 5 for the B signal are used to correct the gap in black balance between the image pickup device 5 for the B signal and the image pickup device 5 for the G signal. In this way, the black balance can be adjusted so as to make the black levels of the R, G and B video signals approximately equal to each other.

Furthermore, according to this embodiment, the A/D converters 7 in the camera head 2 convert the analog video signal into the digital video signal. Then, the resulting digital video signal is transmitted from the camera head 2 to the camera control unit 3. Therefore, a common digital interface can be used, so that the compatibility can be readily assured even when the combination of the camera head 2, the camera cable 4 and the camera control unit 3 is changed.

Furthermore, according to this embodiment, since digital transmission, such as low voltage differential signaling (LVDS), is adopted, the video signal can be multiplexed with a control signal for a switch on the camera head 2 or memory data, for example. Furthermore, a clock signal or a shutter control signal, for example, can be bidirectionally transmitted from the control unit 10 of the camera control unit 3.

Furthermore, according to this embodiment, the camera cable 4 includes the buffer circuit, and therefore, attenuation of the amplitude of the digital signal can be prevented when the cable is long. Thus, the camera cable 4 can be losslessly elongated.

An embodiment of the present invention has been described for illustrative purposes. The scope of the present invention is not limited to the embodiment, and various variations and modifications can be made to suit the application within the scope defined by the claims.

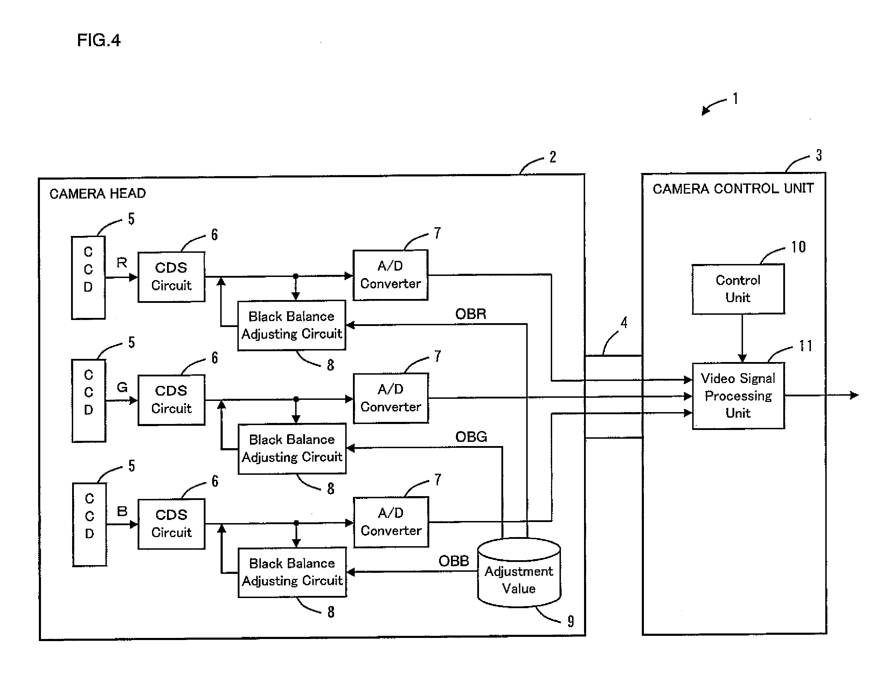

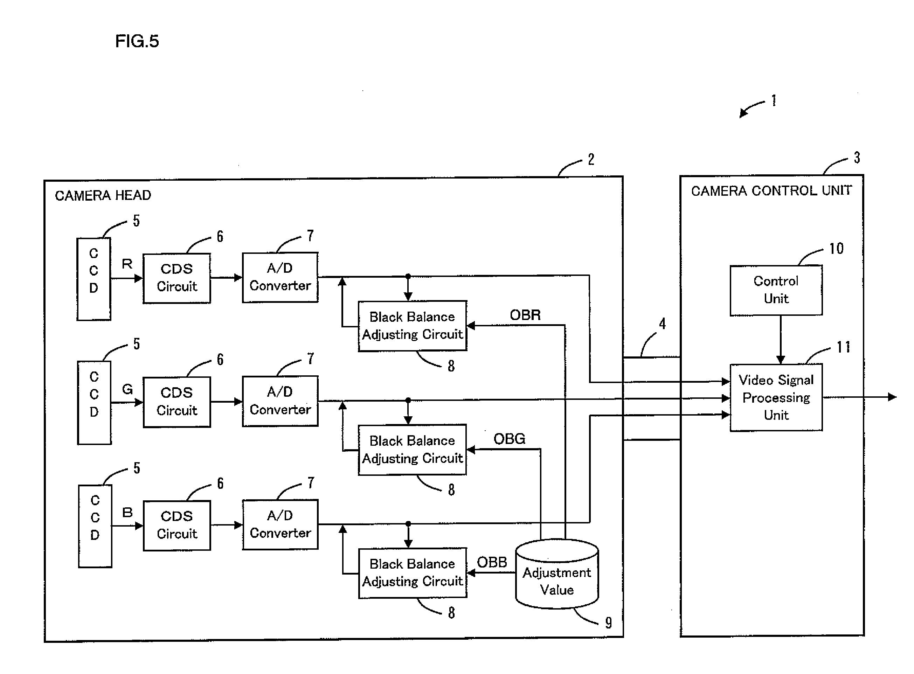

In the example described above, as shown in Figure 1, the black balance adjusting circuits are disposed subsequent to the A/D converters, and the outputs of the black balance adjusting circuits are fed back to the A/D converters. However, the scope of the present invention is not limited to the arrangement. For example, as shown in Figure 4, the black balance adjusting circuits may be disposed preceding the A/D converters. Alternatively, as shown in Figure 5, the outputs of the black balance adjusting circuits may not be fed back to the A/D converters.

Furthermore, in the example described above, the video signals are transmitted from the camera head to the camera control unit in a wired manner (through the camera cable). However, the scope of the present invention is not limited to the arrangement. The video signals may be wirelessly transmitted from the camera head to the camera control unit.

Currently possible preferred embodiments of present invention have been described above. However, it should be understood that various modifications can be made to the embodiments, and all such modifications are included in the scope of the accompanying claims without departing from the true spirit and scope of the present invention.

### Industrial Applicability

As described above, the head separated camera according to the present invention has an advantage that the black balance does not have to be readjusted when the combination of the camera head, the camera cable and the camera control unit is changed and is advantageously used as an endoscope for medical or industrial use.

## Claims

1. A head separated camera in which a camera head is removably attached to a camera control unit that controls said camera head, wherein
said camera head comprises:
a plurality of image pickup devices;
an adjustment value storage unit that stores, as a black balance adjustment value for each of said plurality of image pickup devices, a black balance adjustment value which is set to be specific to said camera head; and
a black balance adjusting unit that adjusts, based on said black balance adjustment value, the black balance of each video signal so as to make the black levels of said video signals produced by said plurality of image pickup devices approximately equal to each other, and
wherein
each video signal of which the black balance is adjusted by said black balance adjusting unit is transmitted from said camera head to said camera control unit.

2. The head separated camera according to Claim 1, wherein
said black balance adjustment value includes:
a reference adjustment value for black balance derived from the video signal from a reference image pickup device, which is one of said plurality of image pickup devices; and
a relative correction parameter for correcting the gap in black balance between said reference image pickup device and each of said plurality of image pickup devices, and wherein
said black balance adjusting unit adjusts, based on said reference adjustment value and said relative correction parameter, the black balance of each video signal so as to make the black levels of said video signals approximately equal to each other.

3. The head separated camera according to Claim 1,
wherein
said camera head further comprises:
an analog-to-digital converting unit that converts said video signal from the analog form to the digital form, and wherein
said video signal is digitally transmitted from said camera head to said camera control unit.

4. A camera head for a head separated camera, comprising:
a plurality of image pickup devices;
an adjustment value storage unit that stores, as a black balance adjustment value for each of said plurality of image pickup devices, a black balance adjustment value which is set to be specific to said camera head; and
a black balance adjusting unit that adjusts, based on said black balance adjustment value, the black balance of each video signal so as to make the black levels of said video signals produced by said plurality of image pickup devices approximately equal to each other.
